# EUROPEAN PATENT APPLICATION

(11) **EP 1 785 728 A1**
(43) Date of publication of application: **16.05.2007**
(21) Application number: 05024949.9
(22) Date of filing: 15.11.2005
(51) Int. Cl.: G01N 33/68, G01N 33/574, A01K 67/027

(54) **Methods and devices for selecting compounds that interact with importin alpha7 and related uses thereof**

(71) Applicant: Max-Delbrück-Centrum für Molekulare Medizin (MDC), 13092 Berlin (DE); Universität zu Lübeck, 23538 Lübeck (DE)
(72) Inventor: Bader, Michael, 13125 Berlin (DE); Shmidt, Tatiana, 13187 Berlin (DE); Hampich, Franziska, 10437 Berlin (DE); Hartmann, Enno, 12683 Berlin (DE); Köhler, Matthias, 24351 Eckernförde (DE)
(74) Representative: Krauss, Jan

(57) **Abstract**

The present invention relates to methods and devices for selecting of compounds that modulate the function of importin α 7, a method for producing a pharmaceutical and contraceptive composition, comprising a modulator, a respective pharmaceutical and contraceptive composition, transgenic non-human animals, and a method for contraception or for the treatment of a proliferative disease, breast cancer, and diabetes or a method of modifying spermatogenesis, comprising administering an effective amount of a modulator of importin α 7.

## Description

The present invention relates to methods and devices for selecting of compounds that modulate the function of importin α 7, a method for producing a pharmaceutical and contraceptive composition, comprising a modulator, a respective pharmaceutical and contraceptive composition, transgenic non-human animals, and a method for contraception or for the treatment of a proliferative disease, breast cancer, and diabetes or a method of modifying spermatogenesis, comprising administering an effective amount of a modulator of importin α 7.

The transport of macromolecules, such as transcription factors, ribonuclear or viral proteins from the cytoplasm through the nuclear pore complexes into the cellular nucleus is an energy dependent process that is triggered by specific signals, such as the "classical" nuclear localization signal (NLS) (for reviews, see Görlich and Kutay 1999, Köhler et al. 1999a, Weis 2003). The following soluble factors that are involved in the classical NLS-dependent import of proteins into the nucleus have been identified until today: importin α, importin β, GTPase Ran/TC4, and NTF2.

Importin is also named "karyopherin" or "pore targeting complex" (PTAC). In the cytoplasm, importin α and importin β form a heterodimeric complex. The importin-complex binds proteins via their NLS and transports them through the nuclear pore, whereby the binding of the NLS-protein through the importin α-subunit leads to the transport of the complex through the nuclear pore occurs via the importin α-subunit. Whereas the formation of the importin-NLS-protein-complex and the transport through the nuclear pore depends on, the release of the imported substrate at the nuclear end of the nuclear pore in dependency of energy and the GTPase Ran. Ran is present in the cytoplasm predominantly in the GDP-loaded version, in the nucleus the GTP-loaded part predominates. Subsequent to the translocation of the import-complex a dissociation of the imported complex occurs through the binding of importin α to Ran-GTP. Whereas the imported substrate can fulfill its function in the nucleus, both importin-subunits are again exported into the cytoplasm, whereby the export of importin α takes place in dependency of Ran-GTP and a protein named CAS.

The nucleo-cytoplasmatic import of substrates bearing a classical NLS is mediated by importin α and β. Six human α importins are described, which can interact with importin β (Cortes et al. 1994, Cuomo et al. 1994, Köhler et al. 1997, Köhler et al. 1999b, Nachury et al. 1998, Seki et al. 1997, Weis et al. 1995). It is yet unclear, why so many isoforms exist. Due to their primary structure, the α-importins can be divided into three subfamilies. Whereas *SRP1* in *S. cerevisiae* encodes for the only and therefore essential importin α-homolog in yeast (Yano et al. 1992), the three subfamilies are also conserved in *D. melanogaster* and *C. elegans.* Investigations on the levels of RNA and protein show that the expression of importin α6 is limited to testis, whereas the other isoforms are diverting in their expression pattern, but in principle are ubiquitary expressed (Köhler et al. 1997, Köhler et al. 1999b). Differences in the regulation of the various isoforms in different models of cellular proliferation and differentiation has recently been shown (Köhler et al. 2002). Several reports describe in vitro substrate specific differences regarding the import efficiency of the different α importins (Sekimoto et al. 1997, Nachury et al. 1998, Köhler et al. 1999b, Welch et al. 1999, Franke et al. 2001, Köhler et al. 2001b). In contrast, only few data exist regarding possible specific roles of individual importin α-isoforms in vivo. For importin α3, a central role for the development of *D. melanogaster* and *C. elegans* has been described (Geles et al. 2001, Mathe et al. 2001). In contrast, the Drosophila homolog of human importin α1 appears to have a function that is essential for oogenesis, whereas its function in the context of the spermatogenesis can be replaced by the other *Drosophila* α-importins (Mason et al. 2002). In vitro data point towards differences in the substrate specific import efficiency of the α-importins. Of the three known α importins in invertebrates, importin α3 appears to be essential for oogenesis and survival of *D. melanogaster* and *C. elegans,* nevertheless, specific functions are not yet elucidated. Similar results regarding the function of the six α importins in vertebrates do not exists. In addition, there is first evidences for tissue specific functions of individual α importins: importin α4 may play a key role in the retrograde transport of signal molecules regulating repair mechanisms in neuronal lesions whereas importin α7 may be involved in the regulation of the development of diabetic nephropathy and tumors.

Köhler et al. (in: Köhler M, Speck C, Christiansen M, Bischoff FR, Prehn S, Haller H, Gorlich D, Hartmann E. Evidence for distinct substrate specificities of importin α family members in nuclear protein import. Mol Cell Biol. 1999 Nov;19(11):7782-91.) describe a novel member of the human importin family, importin α 7. Importin α 7 is the human homologue of the recently identified mouse importin a-S2. To analyze specific functions of the distinct importin a proteins, they recombinantly expressed and purified five human importin α 's along with importin α from *Xenopus* and *Saccharomyces cerevisiae.* Binding affinity studies showed that all importin α proteins from humans or *Xenopus* bind their import receptor (importin beta) and their export receptor (CAS) with only marginal differences. Using an in vitro import assay based on permeabilized HeLa cells, the import substrate specificities of the various importin α proteins were compared.

Hogarth et al (Hogarth C, Itman C, Jans DA, Loveland KL. Regulated nucleocytoplasmic transport in spermatogenesis: a driver of cellular differentiation? Bioessays. 2005 Oct;27(10):1011-25.) examine the importance of nucleocytoplasmic trafficking to male germ cell differentiation, using the sex-determining factors Sry and SOX9, cell cycle regulators, CREM and cofactors and the Smads as specific examples, together with the roles in gametogenesis for particular nuclear transport factors in *Caenorhabditis elegans* and Drosophila. In this study importin α 7 has not been examined.

Furthermore, Loveland et al (Loveland KL, Hogarth C, Szczepny A, Prabhu SM, Jans DA. Expression of Nuclear Transport Importins beta 1 and beta 3 is Regulated During Rodent Spermatogenesis. Biol Reprod. 2005 Sep 28) describe real-time PCR analysis of postnatal mouse testes as revealing changing of expression levels of Knpb1 (encoding importin beta 1) and Ranbp5 (encoding beta 3) mRNAs, with Knpb1 highest at 26 dpp and Ranbp5 highest in day 26 and adult testis. The contrasting expression patterns of importins beta 1 and 3 are proposed candidates for regulating nuclear access of factors required for developmental switches. Importin α 7 has not been examined in this study.

EP 1074617-A, published on February 7, 2001, (Ota, T., Isogai, T., Nishikawa, T., Hayashi, K., Saito, K., Yamamoto, J., Ishii, S., Sugiyama, T., Wakamatsu, A., Nagai, K. and Otsuki, T. Primers for synthesising full-length cDNA and their use; Research Association for Biotechnology (JP)) describes importin α 7. Furthermore, US 6753314-A (22-JUN-2004; Giot, L. and Mansfield, T.A. Protein-protein complexes and methods of using same; CuraGen Corporation), US 6753314 (Sequence 672), US 6890710 (Sequence 12), US 6503703 (Sequence 5), and US 5750394 (Sequence 3) describe proteins similar to importin α 7.

It is therefore an object of the present invention, to provide an improved treatment of proliferative diseases, breast cancer, and/or diabetes or an improved method for contraception on the basis of modulators of importin α 7. It is a further object of the present invention, to identify suitable modulators of importin α 7, and to render them available for such a therapy.

In a first aspect of the present invention, one of these objects of the present invention is solved by a method for identifying modulators of importin α 7. The method comprises the steps of: a) providing a test system, comprising importin α 7 or a biologically active fragment or derivative thereof, b) contacting said test system with one or more compounds suspected of modulating importin α 7, and c) detecting a modulation of importin α 7 by said one or more compounds.

Preferred according to the invention is a method, further comprising the steps of d) identifying of a modulator, and in particular of an inhibitor, of importin α 7 or a biologically active fragment or derivative thereof, and, optionally, e) chemical derivatisation of the modulator identified in step d).

In a second aspect thereof, a further object of the present invention is solved by a method for producing a pharmaceutical and/or contraceptive composition, comprising a) identifying of a modulator, and in particular of an inhibitor, of importin α 7 or a biologically active fragment or derivative thereof according to the present invention, and b) mixing of the modulator, and in particular of the inhibitor, with a suitable pharmaceutical carrier and/or other suitable pharmaceutical auxiliary agents.

A further object of the present invention in a third aspect thereof is solved by a pharmaceutical and/or contraceptive composition, produced according to the present invention, and a compound, identified by means of a method according to the present invention.

A further object of the present invention in a fourth aspect thereof is solved by a transgenic non-human mammal, comprising a functionally inactivated importin α 7. A further object of the present invention in a fifth aspect thereof is solved by a method for contraception, comprising administering an inhibitor of importin α 7 to a mammal. A further object of the present invention in a sixth aspect thereof is solved by the use of a modulator of importin α 7 or a biologically active fragment or derivative thereof for the production of a medicament for the treatment of proliferative diseases, breast cancer or diabetes and the use for male contraception in mammals.

Finally, an additional object of the present invention in a last aspect thereof is solved by a method for treatment of a proliferative disease, breast cancer or diabetes, comprising administering an effective amount of an inhibitor of importin α 7 or a biologically active fragment or derivative thereof to a patient.

The present invention is based on results about the specific functions of selected α importins in the development and the survival of higher eukaryotes. In particular the role of importin α4 for the regenerative growth of injured neurons as well as the role of importin α 7 for the generation of diabetic nephropathy, tumors, and spermatogenesis were examined. By means of in vitro studies on importin α-deficient cells and/or transgenic mice, the specific functions of different α-importins for proliferation and differentiation of human cells have been examined in order to identify new importin α-specific substrates.

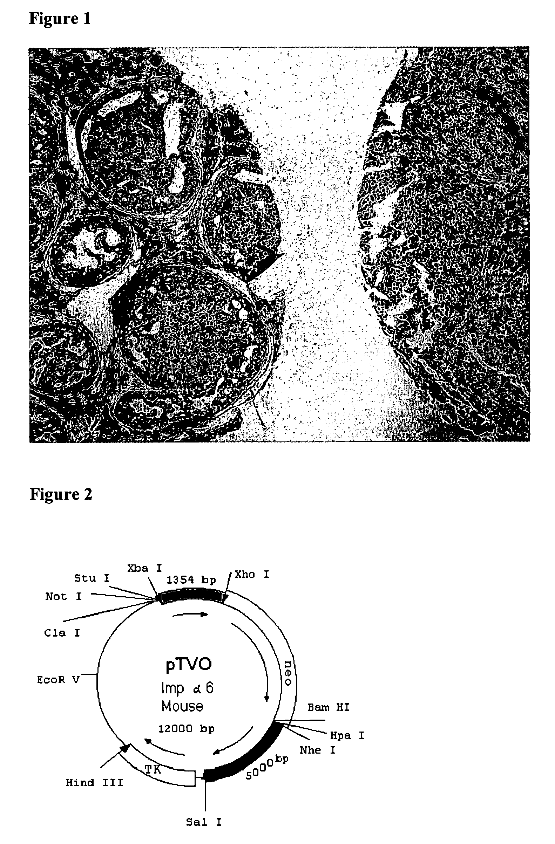

During the experiments in the context of the present invention, evidences for a distorted expression of α importins in different pathological conditions were found:
- In first in vivo analyses for importin α 7 an elevated expression in different diabetic models of the rat as well as in different cultivated cells following stimulation with high glucose concentrations could be shown (Köhler et al. 2001 a). Thus, it is likely that importin α 7 plays an important regulatory role in the generation of diabetic nephropathy.
- In the context of the present invention, it could be shown that importin α 7 is stronger expressed in tissues of breast cancer than in healthy breast tissue (see Figure 1). Also for importin α1 a functional role in the development of breast cancer is discussed (Kim et al. 2000).
- In experiments in the context of the present invention, it was found that the modulation, and in particular the inhibition of importin α 7 in transgenic mice led to an imparted spermatogenesis. Importin α 7 therefore must be regarded as a target for male contraception.

US 2005-0176943 A1 describes mouse spermatogenesis genes, human male sterility-associated genes and a diagnostic system using the same. A transgenic mouse was prepared, wherein the haspin gene was inactivated. Furthermore, it was revealed that there exist at least 8 kinds of intratesticular proteins interacting with haspin molecule. US 2005-0176943 A1 then very generally proposes to cause male infertility by dysfunction of haspin itself or dysfunction of a protein interacting therewith. Importin α is described among molecules interacting with haspin, and dysfunction of the importin α is proposed as inhibiting the transfer of haspin to nucleus and cause functional deficit of haspin. Moreover, the haspin promoter is proposed as potential target to activate the promoter in somatic cells in order to regulate the growth of abnormally growing cells. The activation of the promoter can be achieved by introduction of a specific transcription factor effective thereon or a gene thereof. Alternatively, it can be achieved by a method of activating gene expression of its transcription factor. Nevertheless, US 2005-0176943 A1 does not describe specific importin subunits and in particular no importin α 7 as a binding partner of haspin. Furthermore, it was shown in vitro that the different importin α-proteins have different substrate specificities during nuclear import (Köhler et al. 1999b, Welch et al. 1999, Franke et al. 2001, Köhler et al. 2001b). Thus, the different importin α-proteins exhibit different functions.

The drosophila homolog of human importin α1 appears to be essential for oogenesis but not for spermatogenesis (Mason et al. 2002).

According to the method according to the invention for identifying modulators of importin α 7, a test system that is selected from purified importin α 7, a biologically active fragment or derivative thereof; an importin α 7, a biologically active fragment or derivative thereof expressing cell; an *in vitro* test system; and/or transgenic non-human mammal is used in order to detect a modulation, and in particular an inhibition, of importin α 7 following contacting said test system with one or more compounds suspected of modulating importin α 7 by said one or more compounds.

In a preferred method of the present invention, a modulation, and preferably an inhibition, of the expression and/or the inhibition of the biological activity of importin α 7 or a biologically active fragment or derivative thereof is detected.

The identification of the role of importin α 7 in proliferative diseases, such as breast cancer, diabetes, such as diabetic nephropathy, and in spermatogenesis in the first aspect of the present invention provides the possibility of a use of importin α 7 as a "target" for a method for identifying substances that, directly or indirectly, bind to importin α 7 and modulate, and preferably inhibit, its activity. Methods for routine screenings are well known to the person of skill in the area of pharmaceuticals. By means of high-throughput-technologies suitable compound libraries can be screened and/or searched. These libraries and their screenings are well known to the person of skill, and can readily be modified to be used in the context of the present invention, without the necessity of inventiveness. For example, US 6,821,737 describes methods and kits for screening for transcription factor modulators. Thus, the person of skill will be readily able to adjust the method as described in US 6,821,737 to the present situation.

Preferred according to the present invention is a method, wherein said test system is selected from purified importin α 7, a biologically active fragment or derivative thereof; an importin α 7, a biologically active fragment or derivative thereof expressing cell; an *in vitro* test system; and/or transgenic non-human mammal. Respective test systems are known to the person of skill; these involve, for example, the analysis of the expression of the gene products to be analyzed by means of DNA or RNA-analysis, chip-based analyses, RT-PCR, ELISA or other antibody-based detection methods.

In the context of the present invention, the term "biologically active fragment or derivative thereof" shall mean polypeptides that are functionally related to importin α 7, i.e. share structural features of these polypeptides. Examples of "derivatives" are polypeptides that have a sequence homology, in particular a sequence identity of about 70%, preferably about 80%, in particular about 90%, above all about 95% to the polypeptide with the amino acid sequence of human, mouse or rat, preferably human importin α 7. These derivatives also include additions, inversions, substitutions, deletions, insertions or chemical/physical modifications and/or replacements or parts of the polypeptide in the range of about 1-60, preferably of about 1-30, in particular of about 1-15, above all of about 1-5 amino acids. For example, the first amino acid methionine can be missing, without essentially imparting the biological function of the polypeptide.

In the context of the present invention, the term "modulator" shall mean on the one hand a compound and/or molecule that, directly or indirectly, binds to importin α 7 and influences the biological function of the polypeptide in a positive or negative manner, i.e. have a stimulating or inhibiting effect. Furthermore, a "modulator" in the context of the present invention is a compound and/or molecule that can, directly or indirectly, modulate the expression of the gene for importin α 7 or modulate the translation of importin α 7 in the cells. Preferred is a modulator of importin α 7 or a biologically active fragment or derivative thereof that is selected from chemical compounds of lower molecular weight, peptides, proteins, nucleic acids, antisense-oligonucleotides, and antibodies or fragments thereof.

In the context of the present invention, the term "inhibitor" shall mean on the one hand a compound and/or molecule that, directly or indirectly, binds to importin α 7 and influences the biological function of the polypeptide in a negative manner, i.e. that inhibits the function completely or partially. Thereby, the inhibitor can bind directly to the active centre of importin α 7 or to a position that sterically influences the active centre. Furthermore, the inhibitor can bind in combination with a cofactor, such as, for example, a second chemical group, a peptide, protein, or the like. Furthermore, a "modulator" in the context of the present invention is a compound and/or molecule that can, directly or indirectly, inhibits the expression of the gene for importin α 7 (e.g. as a deletion construct) or inhibits the translation of importin α 7 in the cells. Preferred is an inhibitor of importin α 7 or a biologically active fragment or derivative thereof that is selected from chemical compounds of lower molecular weight, peptides, proteins, nucleic acids, antisense-oligonucleotides, and antibodies or fragments thereof.

A further preferred method of the present invention relates to a method for identifying substances, further comprising a computer-supported structural pre-selection of the one or more compounds suspected of being a modulator of importin α 7 or a biologically active fragment or derivative thereof. By virtual screening substances can be selected that potentially modulate and preferably inhibit importin α 7. These substances are tested, whether they can modulate and preferably inhibit the functioning test. A further preferred embodiment of the methods of the present invention furthermore comprises a computer-based structural pre-selection of the one or more compounds suspected of being a modulator and preferably an inhibitor of importin α 7 or a biologically active fragment or derivative thereof. One particular preferred strategy is the structural analysis of complexes of importin β, importin α 7 with cognate substrates that are compared to complexes with other alpha-importins and the same substrate, or that are compared to complexes of importin β and importin α 7 and a non-cognate substrate in order to obtain information for in silico drug screening or drug design as above. Respective computer-based methods are known to the person of skill.

A further aspect of the present invention relates to a method according to the present invention as above, further comprising the steps of d) identifying of the modulator, and in particular of the inhibitor, of importin α 7 or a biologically active fragment or derivative thereof, and, optionally, e) chemical derivatisation of the modulator and in particular of the inhibitor as identified in step d). If such a modulator and in particular an inhibitor can bed found with the aid of a test according to the present invention, this compound according to the present invention constitutes a lead substance or lead compound for the further commercial development of a medicament. It is then used in subsequent, in particular living, test systems and developed further.

A further preferred embodiment of the method of the present invention furthermore comprises the step of the chemical derivatisation of the compounds selected as above. As used herein, in the context of the present invention a "derivative" shall mean a compound that is derived from the compound as identified according to the present invention which, for example, is substituted with different residues or chemical groups as well as mixtures of several of these compounds which, for example, can be produced into a "personalized" medicament that is adjusted to the disease to be treated and/or the patient on the basis of diagnostic data or data regarding the success of the treatment or its progression. In the context of the present invention a "chemical derivatisation" shall mean a method for a respective chemical modification, that is, for example, the substitution with different residues or chemical groups. Preferably, a chemical derivatisation is performed with the aim of providing an improved bioavailability or a reduction of possible side-effects. In the context of the present invention, a "derivative" shall also mean a "precursor" of a substance that in the course of the administration for a treatment is modified through the conditions inside the body (e.g. pH in the stomach, or the like) or which is metabolized by the body following the uptake in such a manner that a compound according to the present invention or its derivatives is formed as biologically effective substance.

A further aspect of the present invention then relates to a method for producing a pharmaceutical and/or contraceptive composition, comprising a) identifying of a modulator, and in particular of an inhibitor, of importin α 7 or a biologically active fragment or derivative thereof according to the methods as above, and b) mixing of the modulator, and in particular of the inhibitor, with a suitable pharmaceutical carrier and/or other suitable pharmaceutical auxiliary agents.

The production of pharmaceutical and/or contraceptive compositions, e.g. in form of medicaments with a content of modulator, and in particular of the inhibitor, according to the present invention and their uses according to the present invention use occurs according to standard pharmaceutical technology and methods. For this, the modulators, and in particular the inhibitors, together with pharmaceutical acceptable carriers and/or other suitable pharmaceutical auxiliary agents, are produced into medical forms that are suitable for the different indications (and contraception), and places of administration.

Thereby, the medicaments or contraceptive compositions can be produced in a manner that the release rate that is desired, e.g. a quick onset and/or a retard- or depot-effect is achieved. Thereby, the medicament or contraceptive composition can be an ointment, gel, patch, emulsion, lotion, foam, crème or mixed-phase or amphiphilic emulsion systems (oil/water-water/oil-mix-phase), liposome, transfersome, paste or powder.

According to the present invention, the term "auxiliary agent" shall mean any, non-toxic, solid or liquid filling, diluting or packaging material, as long as it does not adversely react and/or interacts with a modulator or the patient. Liquid galenic auxiliary agents, for example, are sterile water, physiological saline, sugar solutions, ethanol and/or oils. Galenic auxiliary agents for the production of tablets and capsules, for example, can contain binders and filling materials.

Furthermore, a modulator according to the invention, and in particular an inhibitor, can be used in the form of systemically employed medicaments. These include parenterals belonging to which are injectables and infusions. Injectables are either present in the form of ampoules or as so-called ready-to-use injectables, e.g. as ready-to-use syringes or disposable syringes, and, in addition, are provided in puncture-sealed bottles. The administration of the injectables can take place in form of subcutaneous (s.c.), intramuscular (i.m.), intravenous (i.v.) or intracutaneous (i.c.) application. In particular the suitable forms for injection can be produced as crystal suspensions, solutions, nanoparticular or colloidal-disperse systems, such as, for example, hydrosoles.

The injectable compositions can further be produced as concentrates that are dissolved or dispersed with aqueous isotonic diluents. The infusions can also be prepared in form of isotonic solutions, fatty emulsions, liposome compositions, micro emulsions. Like the injectables also infusion compositions can be prepared in form of concentrates for dilution. The injectable compositions can also be applied in form of continuous infusions, both in the stationary as well as in the ambulant therapy, e.g. in form of mini pumps.

The modulator according to the invention, and in particular the inhibitor in the parenterals can be bound to a micro carrier or nanoparticle, for example to finely dispersed particles on the basis of poly(meth)acrylates, polylactates, polyglycolates, polyaminoacids or polyetherurethanes. The parenteral compositions can also be modified into a depot preparation, e.g. based on the "multiple unit principle", if a modulator according to the invention, and in particular an inhibitor is embedded in finely divided or dispersed, suspended form or as crystal suspension, or based on the "single unit principle", if a modulator according to the invention, and in particular an inhibitor is included in a medicinal form, e.g. in a tablet or a stick that is subsequently implanted. Often, these implants or depot medicaments in the case of "single unit"- and "multiple unit"-medicaments consist of so-called biodegradable polymers, such as, for example polyesters of lactic and glycolic acid, polyether urethanes, polyaminoacids, poly(meth)acrylates or polysaccharides.

As suitable auxiliary agents for producing of parenterals *aqua sterilisata*, substances influencing the value of the pH, such as, for example, organic and inorganic acids and bases as well as their salts, buffer substances for adjusting the value of the pH, isotoning agent, such as, for example, sodium chloride, sodium hydrogen carbonate, glucose and fructose, tensides or surface active substances and emulgators, such as, for example, partial fatty acid esters of polyoxyethylene sorbitane (Tween®) or, for example, fatty acid esters of polyoxyethylene (Cremophor®), fatty oils, such as, for example, peanut oil, soy bean oil, and castor oil, synthetic fatty acid esters, such as, for example, ethyloleate, isopropylmyristate and neutral oil (Miglyol®), as well as polymeric auxiliary agents, such as, for example, gelatine, dextran, polyvinylpyrrolidone, solubility enhancing additives, organic solvents, such as, for example, propyleneglycol, ethanol, N,N-dimethylacetamide, propylenglycole or complex-forming substances, such as, for example, citrate and urea, preservatives, such as, for example, benzoic acid hydroxypropylesters and -methylesters, benzylalcohol, antioxidants, such as, for example, sodiumsulfite and stabilizators, such as, for example, EDTA, can be considered.

In suspensions, the addition of thickening agents in order to avoid the setting of the modulator according to the invention, and in particular of the inhibitor or the addition of tensides, in order to ensure the admixing of the sediment, or of complex forming agents such as, for example, EDTA is possible. Drug complexes can be achieved with different polymers, such as, for example, polyethyleneglycoles, polystyrenes, carboxymethyl cellulose, Pluronics® or polyethylene glycolsorbite fatty acid esters. For producing lyophilisates, scaffold forming agents, such as, for example, mannit, dextran, sucrose, human albumin, lactose, PVP or gelatines are used.

The medical forms that are each suitable can be produced in accordance with manuals and procedures known to the person of skill on the basis of pharmaceutical/physical technologies.

A further aspect of the present invention then relates to the respectively produced pharmaceutical and/or contraceptive composition. This pharmaceutical composition and/or contraceptive composition can be characterized in that the compound is present in form of a depot substance or as precursor together with a suitable, pharmaceutically acceptable diluent or carrier substance.

Preferred is a pharmaceutical composition according to the present invention, that furthermore contains chemotherapeutics. These chemotherapeutics can comprise all common chemotherapeutics that are known to the person of skill in the context of a cancer therapy (e.g. taxol). According to the present invention, the above pharmaceutical composition can be present in the form of tablets, dragees, capsules, droplets, suppositories, compositions for injection or infusion for peroral, rectal or parenteral use. Such administration forms and their production are known to the person of skill. Particularly preferred is a composition for the administration of an antisense oligonucleotide directed against the expression of importin α 7.

An even further aspect of the present invention then relates to a compound that has been identified (selected) by means of a method according to the present invention. This compound, for example, can be derived from a (commercially available) natural and/or artificial compound library, such compound libraries are well known to the person of skill. Preferred is a library of short peptides. It is particularly preferred that the compound is selected from chemical compounds of lower molecular weight, peptides, proteins, nucleic acids, antisense-oligonucleotides, and antibodies or fragments thereof.

A further aspect of the present invention relates to an oligonucleotide that specifically hybridizes to the nucleic acid sequence of importin α 7. Oligonucleotides represent important therapeutics in gene therapy. The oligonucleotides according to the present invention can be present in the form of nucleic acids comprising DNA, dsDNA, RNA, mRNA, siRNA, PNA and/or CNA. Preferably, the oligonucleotides are present as "antisense"-oligonucleotides. The upper limit for oligonucleotides is determined by the respective practical use, whereby usually a maximal length of 50-200 nucleotides is preferred.

Usually, oligonucleotides are quickly degraded through endo- or exonucleases, in particular through DNases and RNases that can be found in the cell. It is therefore advantageous to modify the nucleic acid in order to stabilize it against degradation, such that a high concentration of the nucleic acid is maintained in the cell over a long period of time (WO 95/11910, WO 98/37240; WO 97/29116, Dudycz 1995, Macadam et al. 1998). Typically, such a stabilization can be obtained through the introduction of one or more internucleotide-phosphate groups or through the introduction of one or more non-phosphor-internucleotides.

Suitably modified internucleotides are summarized in Uhlmann and Peymann, 1990 (see also WO 95/11910, WO 98/37240; WO 97/29116, Dudycz 1995, Macadam et al. 1998). Modified internucleotides-phosphate residues and/or non-phosphoresterbonds in a nucleic acid that can be used in one of the uses according to the present invention, for example, contain methylphosphonates, phosphorothioates, phosphoramidates, phosphorodithioates, phosphateesters, whilst non-phosphorous-internucleotide-analogs, for example siloxane bridges, contain carbonate bridges, carboxymethylesters, acetamidate bridges and/or thiobridges. It is also envisaged that this modification improves the shelf life of a pharmaceutical or contraceptive composition, that can be employed in one of the uses according to the present invention.

Using "antisense"-oligonucleotides the expression of the respective genes of importin α 7 can be reduced in cells both *in vivo* as well as *in vitro.* For the use as "antisense"-oligonucleotide, a single-stranded DNA or RNA is preferred.

In order to allow the introduction of nucleic acids according to the invention as modulators, and in particular inhibitors in the importin α 7 expressing cell by transfection, transformation or infection, the nucleic acid can be present as a plasmid, as part of a viral or non-viral vector.

Suitable as viral vectors are in particular retroviruses, baculoviruses, vaccinia viruses, adenoviruses, adeno-associated viruses and herpes viruses. Suitable as non-viral vectors are in particular virosomes, liposomes, cationic lipids, or polylysine-conjugated DNA.

Examples of vectors effective for gene therapy are viral vectors, for example adenoviral vectors or retroviral vectors, which are well known in the state of the art.

A further matter of the present invention is a modulator and in particular an inhibitor in form of a polyclonal or monoclonal antibodies or an importin α 7-binding fragment thereof, preferred is a monoclonal antibody. The term "antibody" according to the present invention shall also include genetically produced and, optionally, modified antibodies or antigen binding parts thereof, such as, for example, chimeric antibodies, humanized antibodies, multifunctional antibodies, bi- or oligo-specific antibodies, single-stranded antibodies, F(ab)-or F(ab)₂-fragments (see e.g. EP-B1-0 368 684, US 4,816,567, US 4,816,397, WO 88/01649, WO 93/06213, and WO 98/24884).

A further aspect relates to the administration of the modulators, in particular the inhibitors according to the invention in the form of oligonucleotides during gene therapy via common transfection systems, such as, for example, liposome- or "particle gun"-techniques.

Starting from the structures as identified in a preferred embodiment of the method according to the present invention structurally similar substances (derivatives) can be produced that specifically bind to the target importin α 7 in the context of a "molecular mimicry".

A further important aspect of the present invention relates to transgenic non-human mammal, comprising a functionally inactivated importin α 7. Preferably, said transgenic non-human mammal is a rodent, such as a mouse or rat. Said transgenic non-human mammal can be heterozygous or preferably homozygous, and is used as a valuable tool in order to study the functions of importin α 7 or can be used in screenings as above.

A further important aspect of the present invention relates to a method for contraception, comprising administering an effective amount of an inhibitor of importin α 7 to a mammal, in order to inhibit the expression and/or biological function of importin α 7. Preferably, said mammal is a male human. A further important aspect of the present invention relates to the use of a modulator, and in particular an inhibitor according to the invention of importin α 7 or a biologically active fragment or derivative thereof, as defined herein, for the treatment of a proliferative disease, breast cancer or diabetes. Preferred is a use according to the invention, wherein said inhibitor is a pharmaceutical or contraceptive composition as defined herein or a compound as defined herein. Preferred is a use according to the invention, wherein said proliferative disease is breast cancer and said diabetic disease is diabetic nephropathy.

A further important aspect of the present invention relates to a method for the treatment of a proliferative disease, breast cancer or diabetes, comprising administering an effective amount of an inhibitor of importin α 7 or a biologically active fragment or derivative thereof to a patient. Preferred is a method according to the invention, wherein said proliferative disease is breast cancer and said diabetic disease is diabetic nephropathy. The invention relates to the correlation of the biological function of importin α 7 with spermatogenesis and proliferative diseases, such as breast cancer or diabetic diseases. Thus, the use of this gene as a therapeutical target or as a target for contraception is given. According to the present invention such treatments comprise the administration of a pharmaceutical or contraceptive composition as defined herein. A further particular aspect of the present invention therefore relates to a method wherein said proliferative disease is breast cancer and said diabetic disease is diabetic nephropathy and a pharmaceutical composition as defined herein is administered as a medicament.

In another embodiment of the present invention, the medicament that is used according to the present invention, is administered via different routes, such as, for example, orally, parenterally, subcutaneously, intramuscularly, intravenously or intracerebrally. The preferred route of administration would be parenterally in a daily dose of the compound for an adult of about 0.01-5000 mg, preferably 1-1500 mg per day. Preferably, said medicament is administered in a dose of between 30 mg/day and 2000 mg/day, preferably between 100 mg/day and 1600 mg/day, and most preferred between 300 to 800 mg/day. The suitable dose can be presented as a single dose or as divided doses, in suitable intervals, for example, as two, three, four or more subdoses per day. Similar dosages and regimens can be applied for contraception.

Suitable dosages can readily be obtained by the person of skill through routine experimentation, and can be based on factors, such as, for example, the concentration of the active drug, the body weight and age of the patient, and other patient- or active drug-related factors.

Pharmaceutical or contraceptive compositions are generally administered in an amount that is effective for the treatment or prophylaxis of a specific condition or conditions, or for contraception. The initial dose in a human is accompanied by a clinical monitoring of the symptoms, that is, the symptoms of the selected condition (including spermatogenesis). In general, the compositions are administered in an amount an active drug of at least about 100 µg/kg body weight. In most cases, they are administered in one or more dosages in an amount not in excess of about 20 mg/kg body weight per day. In most cases, a dose of about 100 µg/kg to about 5 mg/kg body weight daily is preferred.

In a final aspect of the present, the present invention relates to a kit comprising materials for performing any of the above methods according to the present invention. Preferred is a kit for identifying modulators of importin α 7, comprising a test system, comprising importin α 7 or a biologically active fragment or derivative thereof together with materials and components for detecting a modulation, and in particular an inhibition, of importin α 7 by said one or more compounds that are screened. Preferably, said test system is selected from purified importin α 7, a biologically active fragment or derivative thereof; an importin α 7, a biologically active fragment or derivative thereof expressing cell; an *in vitro* test system; and/or transgenic non-human mammal. Further preferred, the kit comprises means for identifying of the modulator, and in particular of the inhibitor, of importin α 7 or a biologically active fragment or derivative thereof. Respective means as well as buffers, dyes, labels, devices etc. are as described herein and will become readily available to the person of skill upon reading the present specification.

The present invention shall now be described further in the following examples with respect to the attached drawings without being limited thereto. For the purposes of the present invention, all references as cited herein are incorporated by reference in their entireties.
**Figure 1** shows the comparison of the expression of importin α7 in healthy versus tumorous human breast tissue.
**Figure 2** schematically shows the knockout-construct pTV0Impα3 for importin α7.
**Figure 3** shows an example for the detection of two successfully recombined ES cell-clones (2 and 9) for importin α7 by means of PCR. M, DNA-size markers, C, positive control.
**Figure 4** shows the detailed histology of testis of importin α7 -/- 16-week-old male mice.
**Figure 5** shows the detailed histology of testis of wild type 16-week-old male mice.
**Figure 6** shows the histology of seminiferous tubules and epididymides of 16-week-old male mice in the cauda epididymides of importin α7 -/- mice.
**Figure 7** shows the histology of seminiferous tubules and epididymides of 16-week-old male wild type mice in the cauda epididymides.

### Examples

The following table gives an overview of the names and aliases of different importins and their orthologs.

| **Present invention** | **Ortholog in human** | **Ortholog in mouse** |
|---|---|---|
| Importin alpha 1 (Rch1) | karyopherin alpha 2 | pendulin |
| | | karyopherin alpha 2 |
| | hsRP1 | importin alpha P1 |
| | Importin alpha-2 subunit | Importin alpha-2 subunit |
| | Karyopherin alpha-2 subunit | Karyopherin alpha-2 subunit |
| | | SRP1-alpha |
| | | RAG cohort protein 1 |
| | SRP1-alpha | Pore targeting complex 58 kDa subunit) (PTAC58) |
| Importin alpha 2 | importin alpha 1b | |
| Importin alpha 3 | karyopherin alpha 4 | karyopherin alpha 4 |
| | Importin alpha-4 subunit | Importin alpha-4 subunit |
| | Karyopherin alpha-4 subunit | Karyopherin alpha-4 subunit |
| | | Importin alpha Q1 |
| | Qip1protein | |
| Importin alpha 4 Importin alpha 4 | karyopherin alpha 3 | karyopherin alpha 3 |
| | Importin alpha-3 subunit | |
| | Karyopherin alpha-3 | importin alpha Q2 |
| | subunit | Importin alpha-3 subunit |
| | SRP1-gamma | Karyopherin alpha-3 subunit |
| | SRP1-like protein | |
| Importin alpha 5 (SRP1) | karyopherin alpha 1 | karyopherin alpha 1 |
| | Importin alpha-1 subunit | importin alpha S1 |
| | Karyopherin alpha-1 subunit | Importin alpha-1 subunit |
| | | Karyopherin alpha-1 subunit |
| | SRP1-beta | SRP1-beta |
| | RAG cohort protein 2 | RAG cohort protein 2 |
| | Nucleoprotein interactor 1 NPI-1 | Nucleoprotein interactor 1 |
| Importin alpha 6 | karyopherin alpha 5 | n.d. |
| Importin alpha 7 | karyopherin alpha 6 | karyopherin alpha 6 importin alpha S2 |

### Cloning, abundance in tissues, and functional analysis of novel importin α-proteins

In addition to the known human importin α-proteins, importin α1/Rch1 and importin α5/hSRP1, recently, the complete cDNAs of four novel importin α-proteins could be cloned and sequenced (importins α3, α4, α6, and α7, see Köhler et al. 1997, and 1999b). The analysis of the expression of the novel isoforms on the level of RNA in Northern blots showed transcripts for importins α3, α4; and α7 in nearly all examined human tissues with different intensities. In contrast, a transcript for importin α6 could only be detected in testes (Köhler et al. 1997, and 1999b). Via producing peptide specific antibodies an ubiquitary expression of the different importin α proteins could be detected both in Western blots of protein lysates of different human tissues as well as in several human cell lines. However, the intensity of the expression of each importin α-isoform varied depending on the tissue or cell line (K6hler et al. 1999b).

Following recombinant expression and purification; interaction of human importins α1/ Rch1, α3, α4, α5/hSRP1 and α7 with importin α and CAS could first be detected and quantitatively compared (Köhler et al. 1999b). Furthermore, for the first time direct experimental proof of the import function of the newly described importins α3, α4, and α7 could be performed by means of an in vitro import assay (see Adam et al. 1990). By the simultaneous addition of differently labeled substrates into the reaction mix it could further be shown in vitro that the various importin α-proteins have different substrate specificities during nuclear import (Köhler et al. 1999b, Welch et al. 1999, Franke et al. 2001, Köhler et al. 2001b). First examinations regarding the regulation of the α-importins showed that these are differently regulated in different cellular proliferation or differentiation models (Köhler et al. 2002).

During the experiments in the context of the present invention, evidences for a distorted expression of α importins in different pathological conditions were found:
- In first in vivo analyses for importin α7 an elevated expression in different diabetic models of the rat as well as in different cultivated cells following stimulation with high glucose concentrations could be shown (Köhler et al. 2001 a). Thus, it is likely that importin α7 plays an important regulatory role in the generation of diabetic nephropathy.
- In the context of the present invention, it could be shown that importin α7 is stronger expressed in tissues of breast cancer than in healthy breast tissue (see Figure 1). Furthermore, a functional role of importin α1 during the development of breast cancer has been discussed (Kim et al. 2000).
- In experiments in the context of the present invention, it was found that the modulation, and in particular the inhibition of importin α7 in transgenic mice led to an imparted spermatogenesis. Importin α7 therefore must be regarded as a target for male contraception.

In order to thus analyze the role of the individual α importins for the proliferation of living cells, the individual isoforms were specifically inhibited in their expression in HeLa-cells by means of RNA-interference (RNAi). Whereas an inhibition of the expression of importin α4 to less than 10% of the protein level had no effect on the cellular proliferation, and the effects for importin α1 only led to a low inhibition of the cellular proliferation, blocking of the expression of importins α3, α5, α7 or β, respectively, each led to a strong inhibition of cellular proliferation. The mechanisms that lead to this distortion of cellular proliferation through a selective lack of importin α are yet unknown. Without wanting to be bound by theory, it can be assumed that the nuclear translocation of certain substrates is inhibited by the lack of importin α, which, directly or indirectly - are essential for cellular proliferation. In order to prove such a postulated import-defect, the translocation of RCC1 that was injected into the cytoplasm of cultivated cells was examined. It could be shown that nuclear import of RCC1 in HeLa-cells was specifically delayed by the inhibition of the expression of importin α3 by RNA-interference, a result that is also expected with importin α7, an effect that can be reversed by simultaneous injection of recombinant importin α3 or α7 (Quensel et al. 2004).

Recently, it could be shown that α and β importins appear to play an essential role for the retrograde transport of signal molecules of injured neurons (Hantz et al. 2003). It could be shown that importin α4 can bind the motor protein dynein. Then, importin α4 and dynein, in a complex with importin β that is newly synthesized in injured neurons, can transport NLS-containing signal molecules from the site of injury to the cellular soma, where these then regulate the regeneration of the injured neuron. Blocking of α importins by NLS-peptides led to a clear delay of the regenerative outgrowth of injured cultivated neurons. Furthermore, in-vivo application of NLS-peptides to injured sciatic nerves in living rats led to a clear inhibition of the regenerative outgrowth of injured neurons.

### Generation of knockout-constructs for importin α7

Since evidences for specific physiological functions in the context of the overall organism is present for importin α7, this gene was selected in order to generate knockout-mice. Importin α7 is the most recently described human α importin (Köhler et al. 1999b). In the mouse, no homologous form of the human importin α6 is known, such that the mouse homologue of human importin α7 is described as mouse importin α6 in the database. The inhibition of importin α7 leads to a strong inhibition of the HeLa cellular proliferation.

The knockout-construct for α7 (pTV0Impα6) for the introduction into embryonic stem (ES-) cells of the mouse is described herein. The importin α7 knockout-construct contains 1354 bp from the promoter region of the importin α7 gene as short arm and about 5 kb of the intron I as long arm. (Figure 2). Following homologous recombination, the importin α7 knockout-construct results in a deletion of the transcription start, and of the exon 1 of the importin α7 gene.

### Production of murine importin α7 knockout-stem cell clones and heterozygous mice for importin α7-deficiency

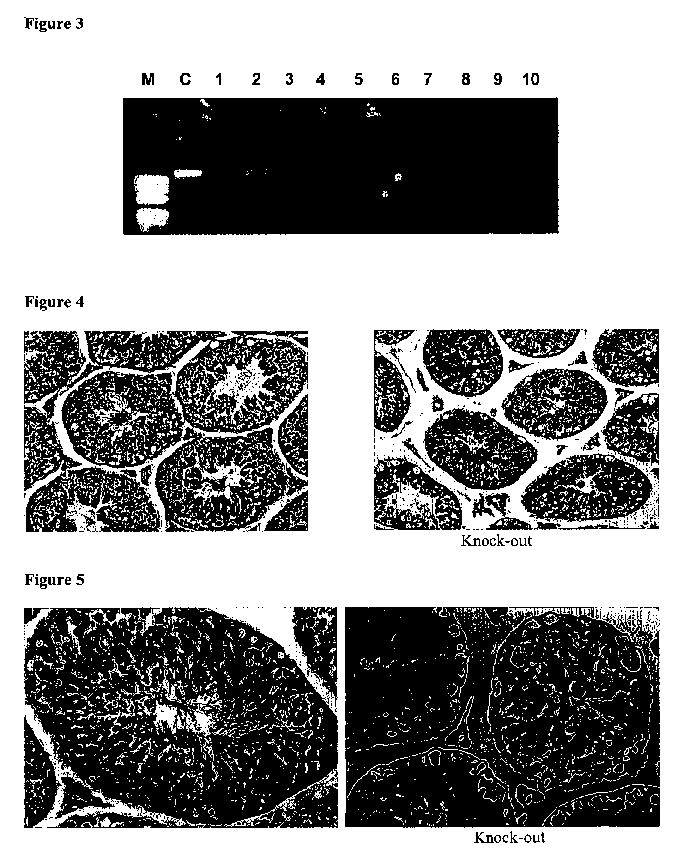

The linearized vector pTV0Impα6 was introduced into ES-cells by electroporation, and following double selection with G418 and gancyclovir 176 resistant clones were obtained. Clones with a correctly recombined homolog genotype were identified through a specific PCR (Figure 3). Five positive clones were isolated. These clones were injected into blastocysts of the mouse strain C57B1/6 in order to obtain chimeras and finally knockout-animals. Following the protocol, chimeric animals for importin α7-deficiency have been obtained. Furthermore, homozygous animals that are defective for importin α7 have been obtained. These animals were infertile and showed an imparted spermatogenesis upon microscopic examination. Meantime, heterozygous and homozygous mice for α4-deficiency have been obtained.

In the context of the present invention, the following experiments will be performed:
- A general analysis of specific in vivo functions of the particular α importin, i.e. importin α 7 in mammals.
- The examination of the function of the selected α importin, importin α 7 in particular pathophysiological conditions, such as proliferative diseases, nephropathy or diabetes.
- The design of cell culturing systems for the analysis of the dependency of the import of selected substrates from the presence of the α importin.
- The analysis of the potential of differentiation of cultivated importin α knockout ES-cells.
- A further analysis of the homozygous mice with defective alleles in importin α7 for an analysis of their ontogenesis, physiology and behavior in certain pathophysiological conditions (Regeneration of neuronal injuries, diabetic nephropathy, p53-dependent tumorigenesis).
- Generation of homozygous defective cells for an analysis of the function of importin α7 during transport of selected substrates. Particularly, an analysis of LEF-1 will be performed.
- Generation of importin α-deficient ES-cells and analysis and comparison of the expression patterns by means of microarray technology before and after differentiation with the expression patterns of normal ES-cells.
- Use of the systems as generated for screening for substances that interfere with the biological function(s) of importin α7.

### Phenotypisation of knockout mice

Both mouse strains with defective importin-genes are first examined based on molecular biological parameters. Using Southern blots the correct genotype is verified, and Northern blots or RT-PCRs are used in order to verify the lack of the importin-mRNAs. In addition, the presence of the protein is confirmed by specific antibodies.

Until today, no phenotype for the deletion of a nuclear transport-factor in higher eukaryotes has been described. As already mentioned importin α5 is essential in yeast. The deletion of importin α3 in *Drosophila melanogaster* is either lethal or leads to an arrest of the oogenesis in the surviving flies (Mathe et al. 2000). Furthermore, for the *C. elegans* homolog of importin α3 a role during embryogenesis and larval development has been described (Geles and Adam 2001). It is therefore surprising that most of the human α importins sharing up to 80% identity can not be replaced in their functions during cellular proliferation (Quensel et al., submitted).

Furthermore, examinations regarding the importance of importin α7 for the pathogenesis of diabetic nephropathy are performed. The inventors could show that importin α7 is up-regulated in kidneys of different rat models (streptocotocin-treatment, Goto-Kakizaki-rats) as well as following incubation with high molar glucose concentration in different cells (Köhler et al 2001 a). This is interpreted as evidence for a possible regulatory role of importin α7 in the development of diabetic nephropathy. Following injection of streptocotocin in importin α7-deficient mice it shall be analyzed if these mice differ from control-animals regarding the development of a diabetic nephropathy (proteinuria, increase of values of retention, comparison of blood glucose levels, histological detection of diabetic nephropathy).

Another pathophysiological question to be analyzed based on the initial experiments is the role of α importins for the progression of the growth of tumors that is examined using the inventive knockout-mice. Kim and co-workers reported the identification of a truncated form of importin α1 in a breast cancer cell line (Kim et al. 2000). They described this truncated importin α-form as responsible for a reduced nuclear translocation of the tumor suppresser p53, and thus responsible for the increased proliferation of breast cancer cells. In contrast thereto, the over-expression of wild-type importin α1 led to an increased cellular apoptosis (Kim et al. 2000). Furthermore, evidences for a possible role of importin α7 in the growth of tumors could be found in the context of the present invention. A clear over-expression of importin α7 in breast cancer tumor tissue when compared to normal mamma tissue could be shown (Figure 3). Mice that carry the dominant negative mutations for p53 are viable but develop a series of spontaneous malign diseases (Donehower et al. 1992, Donehower et al. 1996). In analogy to studies of Wang and co-workers (Wang et al. 2003) the hypothesis shall be examined by crossing to inventive importin α7- and p53-deficient mice, whether importin α7 plays an important functional role in the growth of tumors, and whether a lack of importin α7 leads to a reduced growth of several malign diseases.

### Analysis of importin α-deficient cells

Furthermore, selected cells are obtained from the knockout-mice for which an expression of importin α7 in humans was detected (e.g. lymphocytes, endothelial cells, keratinocytes, mesangial cells) in order to cultivate these or to generate importin α-deficient ES-cells through an additional inactivation of the second allele in the ES-cells using a knockout allele.

In order to test the hypothesis whether a lack of an α importin leads to an inhibition of the nuclear translocation of certain substrates, will be transfection of these knockout-cells with a vector that encodes for the expression of selected GFP-tagged substrates whether the lack of one of the importins leads to a defect of the nuclear transport of the substrates. Alternatively, recombinantly expressed, purified and fluorescein-labeled proteins shall be injected into the knockout-cells, and their subcellular localization will be analyzed by immunofluorescence microscopy.

A series of substrates in form of GFP-encoding vectors for cell-transfection and/or as already purified proteins for cellular injection (RCC1, adenoviral E1A, STAT1, hnRNPK, Egr-1, p53 etc.) are used for first experiments. Of particular interest are two constructs of the Wnt-signaling family, LEF-1 and TCF-1 that are provided from the group of Dr. Waterman, Irvine/CA.

Furthermore, the following questions will be examined in the cultivated importin α knockout ES-cells: 1) viability of these cells 2) general differentiation potential of these ES-cells 3) lack of certain pathways of differentiation in importin α deficient ES-cells. As described by Maltsev et al. in 1994, ES-cells spontaneously differentiate in hanging drops into so-called. embryoid bodies having numerous different cellular types. From these, for example, cardiomyocytes can be selectively obtained. Through microarray technology (Affymetrix), it can be examined on non-differentiated and already differentiated ES-cells, if importin α deficient cells differ from control cells in their gene expressions patterns, and whether, for example, a lack of certain markers points to a lack of the differentiation potential of an importin α deficient ES-cell in a particular cell type. The results of these microarray experiments are then verified by means of RT-PCR on the RNA-level or by means of specific antibodies in Western blot analysis on the protein level. The results of the experiments as performed in the context of the present invention regarding substrate specificity and different regulation of the α importins as well as different effects on cellular proliferation by importin α inhibition propose that the individual isoforms differ essentially in their importance for the regular progression of intracellular signal transduction cascades. It must therefore be expected that the planned microarray experiments will also give important insights into a putative regulatory role of the selected importins for cellular proliferation and cellular differentiation, and will become starting points for further functional analyses.

Finally, the importin α7-deficient cells according to the invention are used to verify the results of other in vitro analyses and to screen for importin activity modulating substances in living cell.

### Arrest of spermatogenesis in importin α7 -/- mice

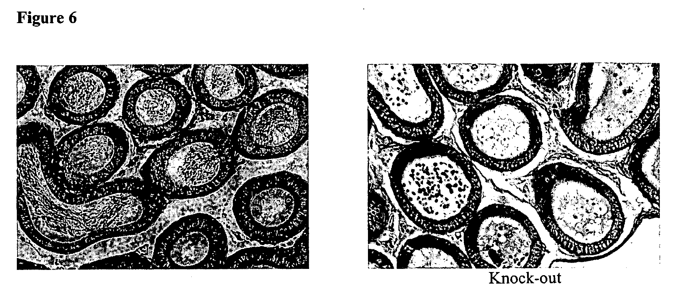

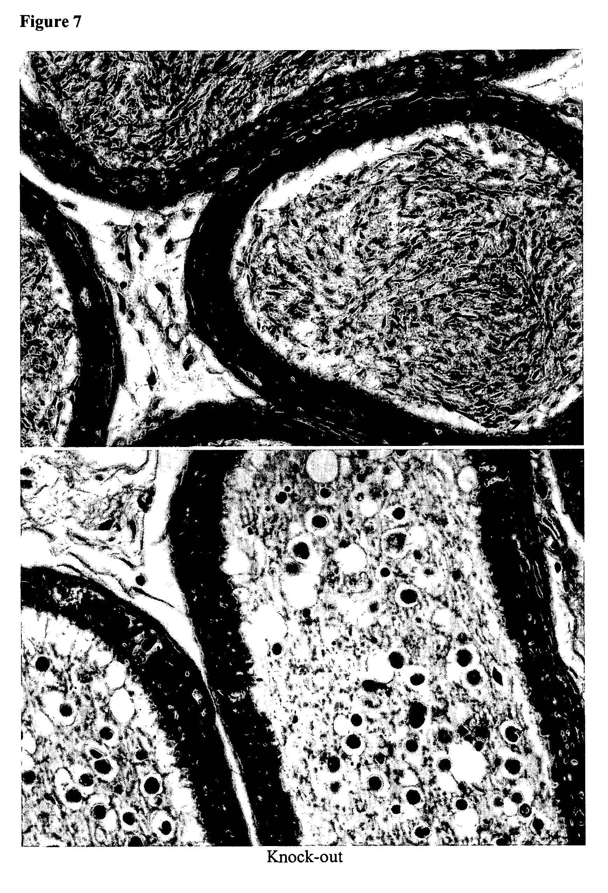

Histology of seminiferous tubules and epididymides of 16-week-old male mice has been performed as follows. Sections were stained with hematoxylin and eosin stain. No sperms could be found in the cauda epididymides of importin α7 -/- mice, in contrast to those of the wild-type mice, which accounts for the infertility of the mutant mice (Figs. 6 and 7). Detailed analysis of the testis revealed that round spermatids failed to undergo normal morphological changes to become elongated spermatids (Figs. 4 and 5). Thus, spermatogenesis is arrested at the stage of spermiogenesis in importin α7 -/- mice.

### Cited literature:

Adam A, Sterne-Marr R, Gerace L: Nuclear protein import in permeabilized mammalian cells requires soluble cytoplasmic factors. J Cell Biol 1990, 111:807-816;
Cortes P, Ye ZS, Baltimore D: RAG-1 interacts with the repeated amino acid motif of the human homologue of the yeast protein SRP1. Proc Natl Acad Sci USA 1994, 91:7633-7637; Cuomo CA, Kirch SA, Gyuris J, Brent R, Oettinger MA: Rch1, a protein that specifically interacts with the RAG-1 recombination-activating protein. Proc Natl Acad Sci USA 1994, 91: 6156-6160;
Donehower LA, Harvey M, Slagle BL, McArthur MJ, Montgomery CA Jr., Butel JS, Bradley A: Mice deficient for p53 are developmentally normal but susceptible to spontaneous tumours. Nature 1992, 356: 215-221;
Donehower LA: The p53-deficient mouse: a model for basic and applied cancer studies. Semin Cancer Biol 1996, 7: 269-278;
Franke J, Reimann B, Hartmann E, Köhler M, Wiedmann B: Evidence for a nuclear passage of nascent poly-peptide-associated complex subunits in yeast. J Cell Sci 2001, 114: 2641-2648;
Geles KG, Adam SA: Germline and developmental roles of the nuclear transport factor importin α3 in C. elegans. Development 2001, 128: 1817-1830;
Görlich D, Kutay U: Transport between the cell nucleus and the cytoplasm. Annu. Rev. Cell Dev Biol 1999, 15: 607-660;
Hanz S, Perlson E, Willis D, Zheng JQ, Massarwa R, Huerta JJ, Koltzenburg M, Kohler M, van-Minnen J, Twiss JL, Fainzilber M: Axoplasmic importins enable retrograde injury signaling in lesioned nerve. Neuron 2003, 40: 1095-1104;
Kim I, Kim D, Han S, Chin M, Nam H, Cho H, Choi S, Song B, kim E, Bae Y, Moon Y: Truncated form of importin α identified in breast cancer cell inhibits nuclear import of p53. J Biol Chem 2000, 275: 23139-23145;
Köhler M, Ansieau S, Prehn S, Leutz A, Haller H, Hartmann E: Cloning of two novel importin-α subunits and analysis of the expression pattern of the importin- α protein family. FEBS Lett 1997, 417: 104-108;
Köhler M, Buchwalow IB, Alexander G, Christiansen M, Shagdarsuren E, Samoilova V, Hartmann E, Meervala, EMA, Haller H: Increased Importin α Protein Expression in Diabetic Nephropathy. Kidney Int 2001a, 60: 2263 - 2273;
Köhler M, Fiebeler A, Hartwig M, Thiel S, Prehn S, Kettritz R, Luft FC, Hartmann E: Differential expression of classical nuclear transport factors during cellular proliferation and differentiation. Cell Physiol Biochem 2002, 12: 335-344;
Köhler M, Görlich D, Hartmann E, Franke J: Adenoviral E1A protein nuclear import is preferentially mediated by importin α3 in vitro. Virology 2001b, 289: 186 - 191;
Köhler M, Haller H, Hartmann E: Nuclear protein transport pathways. Exp Nephrol 1999a , 7: 290-294;
Köhler M, Speck C, Christiansen M, Bischoff FR, Prehn S, Haller H, Görlich D, Hartmann E: Evidence for distinct substrate specifities of importin α-family members in nuclear protein import. Moll Cell Biol 1999b, 19: 7782-7791;
Maltsev VA, Wobus AM, Rohwedel J, Bader M, Hescheler J: Cardiomyocytes differentiated in vitro from embryonic stem cells developmentally express cardiac-specific genes and ionic currents. Circ Res 1994, 75:233-244;
Mason DA, Fleming RJ, Goldfarb DS: Drosophila melanogaster importin α1 and α3 can replace importin α2 during spermatogenesis but not oogenesis. Genetics 2002, 161: 157-70;
Mathe E, Bates H, Huikeshoven H, Deak P, Glover DM, Cotterill S: Importin-α 3 is required at multiple sta-ges of Drosophila development and has a role in the completion of oogenesis. Dev Biol 2000, 223: 307-322;
Morano IL, Chai GX, Baltas LG, Lamounier-Zepter V, Lutsch G, Kott M, Haase H, Bader M. Smooth muscle contraction without smooth muscle myosin. Nat Cell Biol 2000, 2: 371-375; Nachury MV, Ryder UW, Lamond AI, Weis K: Cloning and characterization of hSRP1 gamma, a tissue-specific nuclear transport factor. Proc Natl Acad Sci USA 1998, 95: 582-587;
Pesquero JB, Araujo RC, Heppenstall PA, Stucky CL, Silva J-AJr, Walther T, Oliveira SM, Pesquero JL, Paiva AC, Calixto JB, Lewin GR, Bader M. Hypoalgesia and altered inflammatory responses in mice lacking kinin B1 receptors. Proc Natl Acad Sci USA 2000; 97: 8140-8145;
Quensel C, Friedrich B, Sommer T, Hartmann E, Köhler M. In vivo analysis of importin □ proteins reveals cellular proliferation inhibition and substrate specificity. Moll Cell Biol 2004; 24: 10246-10255;
Seki T, Tada S, Katada T, Enomoto T: Cloning of a cDNA encoding a novel importin-α homologue, Qip1 discrimination of Qip1 and Rch1 from hSrp1 by their ability to interact with DNA helicase Q1/RecQL. Biochem Biophys Res Commun 1997, 234: 48-53;
Sekimoto T, Imamoto N, Nakajima K, Hirano T, Yoneda Y: Extracellular signal-dependent nuclear import of Stat1 is mediated by nuclear pore-targeting complex formation with NPI-1, but not Rch1. EMBO J 1997, 16: 7067-7077;
Walther T, Balschun D, Voigt J-P, Fink H, Zuschratter W, Birchmeier C, Ganten D, Bader M. Sustained long-term potentiation and anxiety in mice lacking the Mas protooncogene. J Biol Chem 1998, 273: 11867-11873;
Wang Y, Zhang Z, Kastens E, Lubet RA, You M: Mice with alterations in both p53 and Ink4a/Arf display a striking increase in lung tumor multiplicity and progression: Differential chemopreventive effect of budenoside in wild-type and mutant A/J mice. Cancer Res 2003, 63: 4389-4395;
Weis K, Mattaj IW, Lamond AI: Identification of hSRP1 α as a functional receptor for nuclear localization sequences. Science 1995, 268: 1049-53;
Walther D, Peter JU, Bashammakh S, Hörtnagl H, Voits M, Fink H, Bader M. Synthesis of serotonin by a second tryptophan hydroxylase isoform. Science 2003, 299: 76;
Weis K: Regulating access to the genome: nucleocytoplasmic transport throughout the cell cycle. Cell 2003, 112: 441-451;
Welch K, Franke J, Köhler M, Macara IA: RanBP3 contains an unusual nuclear localization signal that is imported preferentially by importin α3. Mol Cell Biol 1999, 19: 8400-8411; Xia H, Mao Q, Paulson HL, Davidson B: siRNA-mediated gene silencing in vitro and in vivo. Nat Biotechnol 2002, 20: 1006-1110;
Yano R, Oakes M, Yamaghishi M, Dodd JA, Nomura M: Cloning and characterization of SRP1, a suppressor of temperature-sensitive RNA polymerase I mutations in Saccharomyces cerevisiae. Mol Cell Biol 1992, 12: 5640-5651.

## Claims

1. Method for identifying modulators of importin α 7, comprising the steps of:
a) providing a test system, comprising importin α 7 or a biologically active fragment or derivative thereof,
b) contacting said test system with one or more compounds suspected of modulating importin α 7, and
c) detecting a modulation of importin α 7 by said one or more compounds.

2. Method according to claim 1, wherein said test system is selected from purified importin α 7, a biologically active fragment or derivative thereof; an importin α 7, a biologically active fragment or derivative thereof expressing cell; an *in vitro* test system; and/or transgenic non-human mammal.

3. Method according claim 1 or 2, wherein said modulator of importin α 7 or a biologically active fragment or derivative thereof is selected from chemical compounds of lower molecular weight, peptides, proteins, nucleic acids, antisense-oligonucleotides, and antibodies or fragments thereof.

4. Method according to any of claims 1 to 3, wherein said modulator of importin α 7 or a biologically active fragment or derivative thereof is an inhibitor of importin α 7 or a biologically active fragment or derivative thereof.

5. Method according to claim 4, wherein the inhibition of the expression and/or the inhibition of the biological activity of importin α 7 or a biologically active fragment or derivative thereof is detected.

6. Method according to any of claims 1 to 5, further comprising a computer-based structural pre-selection of the one or more compounds suspected of being a modulator of importin α 7 or a biologically active fragment or derivative thereof.

7. Method according to any of claims 1 to 6, further comprising the steps of
d) identifying of the modulator, and in particular of the inhibitor, of importin α 7 or a biologically active fragment or derivative thereof, and, optionally,
e) chemical derivatisation of the modulator as identified in step d).

8. Method for producing a pharmaceutical and/or contraceptive composition, comprising
a) identifying of a modulator, and in particular of an inhibitor, of importin α 7 or a biologically active fragment or derivative thereof according to any of claims 1 to 7, and
b) mixing of the modulator, and in particular of the inhibitor, with a suitable pharmaceutical carrier and/or other suitable pharmaceutical auxiliary agents.

9. Pharmaceutical and/or contraceptive composition, produced according to claim 8.

10. Compound, identified by means of a method according to any of claims 1 to 7.

11. Compound according to claim 10, selected from chemical compounds of lower molecular weight, peptides, proteins, nucleic acids, antisense-oligonucleotides, and antibodies or fragments thereof.

12. A transgenic non-human mammal, comprising a functionally inactivated importin α 7.

13. A method for contraception, comprising administering an inhibitor of importin α 7 to a mammal.

14. Method according to claim 13, wherein said mammal is a male human.

15. Use of a modulator of importin α 7 or a biologically active fragment or derivative thereof for the production of a medicament for the treatment of proliferative diseases, breast cancer or diabetes.

16. Use of a modulator of importin α 7 or a biologically active fragment or derivative thereof for male contraception in mammals.

17. Use according to claim 15 or 16, wherein said modulator is selected from an inhibitor of importin α 7 or a biologically active fragment or derivative thereof.

18. Use according to any of claims 15 to 17, wherein said inhibitor is a pharmaceutical or contraceptive composition according to claim 9 or a compound according to claim 10 or 11.

19. Method for treatment of a proliferative disease, breast cancer or diabetes, comprising administering an effective amount of an inhibitor of importin α 7 or a biologically active fragment or derivative thereof to a patient.
